# EUROPÄISCHE PATENTANMELDUNG

(11) **EP 3 292 877 A1**
(43) Veröffentlichungstag der Anmeldung: **14.03.2018**
(21) Anmeldenummer: 16188149.5
(22) Anmeldetag: 09.09.2016
(51) Int. Cl.: A61L 27/30, A61L 27/32

(54) **IMPLANTAT ODER OSTEOSYNTHESE UND VERFAHREN ZU DESSEN HERSTELLUNG**

(71) Anmelder: Universität Basel, 4003 Basel (CH); Empa Swiss Federal Laboratories for Materials Science and Technology, 8600 Dübendorf (CH)
(72) Erfinder: Thorwarth, Kerstin, 8600 Dübendorf (CH); Stübinger, Stefan, 4102 Binningen, BL (CH); Patscheider, Jörg, 8706 Meilen (CH)
(74) Vertreter: Kleine, Hubertus

(57) **Zusammenfassung**

Ein Verfahren zur Herstellung eines Implantats oder einer Osteosynthese mit einem nichtmetallischen Substratkörper (55) und einer darauf zumindest bereichsweise angeordneten mehrschichtigen Beschichtung (51-54) umfassend eine metallische Schicht (53) und eine metalloxid- und/oder metallhydroxyapatithaltige Schicht (51), gekennzeichnet durch die folgenden Schritte:
a. Bereitstellen des nichtmetallischen Substratkörpers (Verfahrensschritt I);
b. Aufbringen der metallischen Schicht (53) der mehrschichtigen Beschichtung nach dem HiPIMS-Verfahren (Verfahrensschritt IV); und
c. Aufbringen der metalloxid- und/oder metallhydroxyapatithaltigen Schicht (51) auf die metallische Schicht (53) (Verfahrensschritt VI) durch Sputtern und
ein Implantat oder Osteosynthese umfassend einen Substratkörper (55) aus einem nichtmetallischen Material und eine mehrschichtige Beschichtung (51-54),

## Beschreibung

Die vorliegende Erfindung betrifft ein Verfahren zur Herstellung eines Implantats oder einer Osteosynthese nach dem Oberbegriff des Anspruchs 1 und ein Implantat oder eine Osteosynthese.

Die europäische Anmeldung mit dem Aktenzeichen EP 16 159 953.5 offenbart ein aus mehreren Teilelementen zusammensetzbares Implantat oder eine zusammensetzbare Osteosynthese, welche patientenspezifisch gefertigt ist und im Rahmen eines minimalinvasiven chirurgischen Eingriffs verwendet werden kann. Die jeweiligen Teilelemente können aus einer Vielzahl von Materialien hergestellt werden.

Im Medizinbereich erfolgt oftmals die Verwendung von Titanimplantaten. Diese haben für den Verwendungszweck bevorzugte mechanische Eigenschaften, beispielsweise osseointegrative oder bevorzugtes Knochenanwachsverhalten, und sind chemisch unbedenklich gegenüber Korrosion und dergleichen. Problematisch ist dabei jedoch, dass bestimmte Untersuchungen z.B. MRT oder CT (Computertomographie) zur Überprüfung des chirurgischen Eingriffs nach dem erfolgten Einsatz des metallischen Implantats nur eingeschränkt möglich sind.

Die WO 2015/038489 A1 löst diese Problematik durch das Aufbringen einer dünnen Titanschicht auf einen nichtmetallischen Substratkörper, welcher das Implantat bildet.

Die geringe Schichtdicke und die zugleich gute Anbindung bei einer gleichmäßig guten Verteilung der Metallschicht über die Oberfläche des Substratkörpers wird durch das sogenannte HiPIMS-Verfahren, einem Hochleistungsimpulsmagnetron-Sputtern, erreicht. Durch dieses besondere Sputterverfahren wurden bislang Titan-Schichtdicken erreicht, dass das Implantat oder die Osteosynthese einerseits gegenüber dem anwachsenden Knochengewebe metallisch erscheint und andererseits diese metallische Schicht derart dünn ist, dass auch verschiedene Untersuchungsmethoden zur Überprüfung des chirurgischen Eingriffs möglich sind.

Ausgehend von diesem Verfahren als Stand der Technik ist es nunmehr Aufgabe der vorliegenden Erfindung ein weiter optimiertes Verfahren zur Herstellung eines Implantats oder einer Osteosynthese, sowie ein entsprechendes Implantat oder eine Osteosynthese bereitzustellen.

Die vorliegende Erfindung löst diese Aufgabe durch ein Verfahren mit den Merkmalen des Anspruchs 1 und durch ein Implantat oder eine Osteosynthese mit den Merkmalen des Anspruchs 11.

Ein erfindungsgemäßes Verfahren zur Herstellung eines Implantats oder einer Osteosynthese mit einem nichtmetallischen Substratkörper und einer darauf zumindest bereichsweise angeordneten mehrschichtigen Beschichtung umfassend eine metallische Schicht und eine metalloxid- und/oder metall-hydroxyapatithaltige Schicht. Das Verfahren ist gekennzeichnet durch die folgenden Schritte:
a. Bereitstellen des nichtmetallischen Substratkörpers;
b. Aufbringen der metallischen Schicht der mehrschichtigen Beschichtung nach dem HiPIMS-Verfahren; und
c. Aufbringen der metalloxid- und/oder metall-hydroxyapatithaltigen Schicht auf die metallische Schicht durch ein Sputterverfahren.

Das Bereitstellen des nichtmetallischen Substratkörpers kann patientenspezifisch erfolgen. Dabei kann der Substratkörper auch lediglich ein Teilelement des Implantats oder der Osteosynthese sein. In diesem Fall sind mehrere der Teilelemente zu einem Implantat oder einer Osteosynthese zusammensetzbar. Die Zusammensetzung der Teilelemente selbst, welche nicht Teil des erfindungsgemäßen Verfahrens ist, kann während eines chirurgischen Eingriffs, vorzugsweise minimal invasiv, erfolgen.

Das Aufbringen der metallischen Schicht erfolgt nach dem sogenannten HiPIMS-Verfahren, welches u.a. auch schon aus der WO 2015/038489 A1 bekannt ist. Durch dieses Verfahren lassen sich metallische Schichten mit geringer Schichtdicke auf einen Substratkörper, z.B. aus Kunststoff aufbringen. Diese metallischen Schichten zeichnen sich überdies durch eine im Vergleich zu anderen Sputtermethoden bessere Anhaftung auf dem Substrat aus und durch eine sehr gute Verteilung der Metallschicht auf der Substratoberfläche, insbesondere auch in Kavitäten.

Das Aufbringen einer metallhydroxyapatithaltigen Schicht ist aus der WO 2015/038489 A1 bekannt, allerdings erfolgt hier der Schichtauftrag in einem nasschemischen Verfahren durch Lagerung in einer simulierten Körperflüssigkeit. Diese Schicht ermöglicht durch Einstellung der Porosität einen spannungsarmen Übergang von der metallischen Schicht auf die Hydroxyapatitschicht, welche ihrerseits dasselbe Material wie die mineralische Knochensubstanz darstellt. Dies kann im vorliegenden Fall vorzugsweise durch Reaktivsputtern unter Sauerstoffeinfluss erfolgen.

Vorteilhafte Ausgestaltungen des Verfahrens sind Gegenstand der Unteransprüche.

Das Bereitstellen des nichtmetallischen Substratkörpers kann vorteilhaft die folgenden Schritte umfasst:
a.1 Erfassen von Daten eines Patienten, für welchen das Implantat und/oder die Osteosynthese bestimmt ist
a.2 Erstellen von Fertigungsvorgaben für das Implantat und/oder die Osteosynthese mittels eines Modells anhand der erfassten Daten;
a.3 Fertigung des Substratkörpers auf Basis der Fertigungsvorgaben.

Dadurch kann ein auf den Patienten abgestimmtes Implantat oder eine auf den Patienten abgestimmte Osteosynthese gefertigt werden.

Das Erstellen von Fertigungsvorgaben kann vorteilhaft für zumindest zwei oder mehr zu einem Implantat und/oder zu einer Osteosynthese zusammensetzbare Teilelemente auf Grundlage des erstellten Modells erfolgen, wobei die Fertigungsvorgaben umfassen:
C1) eine Dimensionierung der Teilelemente,
und wobei zumindest eines, vorzugsweise jedes, dieser Teilelemente als Substratkörper ausgebildet ist.

Das Implantat und/oder die Osteosynthese können somit mehrteilig aufgebaut sein. Neben der Dimensionierung der Teilelemente können auch je nach patientenspezifischer Anforderung an die Teilelemente und deren Verbindungen miteinander verschiedene Verbindungstypen ausgewählt werden. Es können zudem auch verschiedene Materialien, z.B. abbaubare Materialien oder Materialien, welche die Heilung unterstützen, für bestimmte Teilelemente genutzt werden. Sofern Letzteres der Fall ist, so kann es von Vorteil sein, wenn dieses Teilelement nicht mit einer metallischen Beschichtung nach dem erfindungsgemäßen Verfahren versehen wird. Einzelne Teilelemente können zudem Sensoren, z.B. MEMS-Sensoren, und/oder mechanische und/oder elektronische Aktuatoren aufweisen, so dass das Implantat oder die Osteosynthese bestimmte Maßnahmen, z.B. Messungen oder Regulierungen im Körper, vornehmen kann.

Zur besseren Anhaftung der metallischen Schicht kann das Bereitstellen des nichtmetallischen Substratkörpers eine Oberflächenreinigung und/oder ein Oberflächenpolieren umfassen.

Ebenfalls zur Verbesserung der Oberflächengüte des Substratkörpers kann nach dem Bereitstellen des nichtmetallischen Substratkörpers und vor dem Aufbringen der metallischen Schicht eine physikalische oder physikalisch-chemische Vorbehandlung der Oberfläche des Implantat- oder Osteosynthese-Substratkörpers durch eine Wärmebehandlung und/oder ein Ätzen erfolgen. Das Ätzen kann ein chemisches oder ein physikalisches Ätzen, beispielsweise eine PlasmaVorbehandlung, sein.

Nach dem Bereitstellen des nichtmetallischen Substratkörpers und vor dem Aufbringen der metallischen Schicht kann vorteilhaft eine physikalische oder physikalisch-chemische Vorbehandlung der Oberfläche des Implantat- oder Osteosynthese-Substratkörpers durch Plasmaaktivierung und/oder durch Aktivierung mit einem Sauerstoffderivat und/oder einer radikalischen Verbindung erfolgt.

Die radikalische Verbindung kann vorzugsweise Ozon sein und die Aktivierung kann als Ozonierung erfolgen. Die Plasmaaktivierung kann beispielsweise durch ICP-Plasmaaktivierung, Mikrowellen-Plasmaaktivierung oder durch Hochfrequenz-Plasmaaktivierung erfolgen. Die Plasmaaktivierung oder die Ozonierung trägt zu einer optimalen Haftung der metallischen Schicht auf dem nichtmetallischen Substrat bei.

Nach dem Bereitstellen des nichtmetallischen Substratkörpers und vor dem Aufbringen der metallischen Schicht nach dem HiPIMS Verfahren kann ein Abscheiden von Metall auf dem Substratkörper durch Sputtern, insbesondere durch Hochfrequenz-Sputtern, erfolgen. Dadurch wird eine elektrisch leitende Schicht auf dem Substrat, auch Target genannt, bereitgestellt, an welche eine sogenannte Bias-Spannung angelegt werden kann.

Nach dem Aufbringen der metallischen Schicht kann ein Aufbringen einer porösen metallischen Schicht, insbesondere durch DC-Sputtern, erfolgen. Die poröse metallische Schicht verringert Ablösetendenzen einzelner Schichten der Beschichtung aufgrund von Ausdehnungen und Materialspannungen. Die Porosität der Schicht wird im Rahmen der vorliegenden Erfindung durch deren Dichte charakterisiert, welche zwischen 50 bis 95% der theoretischen Dichte für das Metall dieser metallischen Schicht liegt.

Nach dem Aufbringen der porösen metallischen Schicht kann ein Aufbringen der metalloxid- und/oder metallhydroxyapatithaltigen Schicht auf die poröse metallische Schicht erfolgen oder eine Ausbildung der metalloxid- und/oder metallhydroxyapatithaltigen Schicht auf der porösen metallischen Schicht erfolgen. Dies kann vorzugsweise durch ein DC-Reaktivsputtern erfolgen.

Ein erfindungsgemäßes Implantat oder Osteosynthese umfasst einen Substratkörper aus einem nichtmetallischen Material und eine mehrschichtige Beschichtung, wobei eine metallische Schicht dieser Beschichtung nach dem HiPIMS-Verfahren aufgebracht wurde. Der mehrschichtige Aufbau der Beschichtung erlaubt zusätzliche Funktionalitäten, z.B. eine Einfärbung des Substratkörpers, bei zugleich dünner Schichtdicke.

Eine metallische Schicht welche nach dem HiPIMS-Verfahren aufgebracht wurde ist gegenüber Schichten die mit anderen Sputterverfahren oder anderen Auftragsverfahren unverwechselbar, aufgrund der erreichbaren Dichte bei gleichzeitiger Erhaltung der vorgängig auf dem Substrat aufgebrachten Oberflächenstrukturierung wie sehr glatte oder spezifische Topographien. Der geringe Wärmeeintrag wä$hrend des Beschichtungsprozesses gegenüber dem gebräuchlichen Plasma-Spritzverfahren ermöglicht das Erzielen dieser vorteilhaften Eigenschaften.

Eine Schicht der Beschichtung, insbesondere die äußerste Schicht, ist vorteilhaft als eine metalloxid- und/oder hydroxyapatithaltige Schicht ausgebildet.

Eine Schicht zwischen der metallischen Schicht, die nach dem HiPIMS-Verfahren aufgebracht wurde, und der metalloxid- und/oder hydroxyapatithaltigen Schicht kann als eine poröse metallische Schicht ausgebildet sein mit einer Dichte, welche zwischen 50 bis 95% der theoretischen Dichte für das Metall dieser metallischen Schicht liegt.

Der nichtmetallische Substratkörper kann vorzugsweise als ein Kunststoffkörper ausgebildet sein, welcher Kunststoff vorzugsweise ausgesucht aus der Klasse der Polyetherketone, Polyetherketonketone, der Polyethylene und/oder der Polyoxymethylene. Der Substratkörper kann vorzugsweise in einem 3-D Druck hergestellt sein. Ebenfalls vorzugsweise sollte der Substratkörper zumindest bis 120°C formstabil sein.

Das Metall der metallischen Schicht und/oder der porösen metallischen Schicht kann vorzugsweise Titan und/oder Tantal sein und das Metalloxid und/oder das Metall-hydroxyapatit kann vorzugsweiseeine Titan- und/oder Tantalverbindung sein.

Nachfolgend wird die Erfindung anhand mehrerer Figuren und Ausführungsbeispiele näher erläutert. Diese sind jedoch nicht einschränkend zu verstehen sondern dienen der näheren Erläuterung der Erfindung. Es zeigen:
- Fig. 1: schematische Darstellung eines Ablaufdiagramms einer Variante eines erfindungsgemäßen Verfahrens zur Herstellung eines mehrteiligen Implantats oder einer mehrteiligen Osteosynthese;
- Fig. 2: schematische Darstellung einer ersten Verbindungsvariante zwischen Teilelementen eines Implantats oder einer Osteosynthese;
- Fig. 3: schematische Darstellung einer zweiten Verbindungsvariante zwischen Teilelementen eines Implantats oder einer Osteosynthese;
- Fig. 4: Ablaufdiagramm eines Verfahrens zur Bereitstellung eines mehrteiligen Implantats oder einer mehrteiligen Osteosynthese; und
- Fig. 5: Schichtaufbau einer metallischen Schicht einer mehrteiligen Implantats oder einer mehrteiligen Osteosynthese.

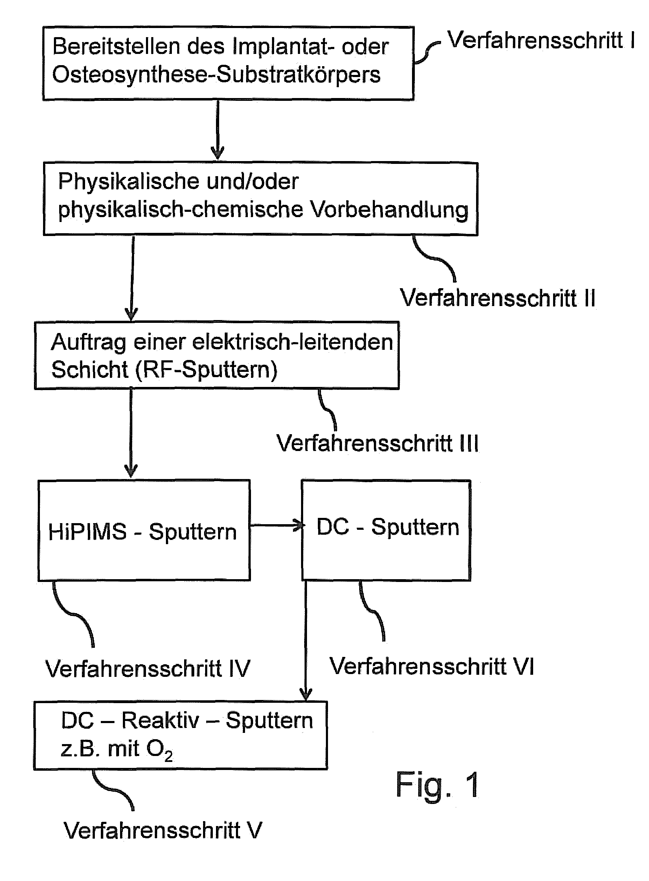

Fig. 1 offenbart einen Verfahrensablauf zur Herstellung eines chirurgischen Implantats.

In einem ersten Verfahrensschritt I erfolgt das Bereitstellen eines Implantat- oder Osteosynthese-Substratkörpers. Dieser Implantat- oder Osteosynthese-Substratkörper kann einstückig, also als Implantat oder Osteosynthese, oder besonders bevorzugt mehrstückig, also als Teilelemente eines Implantats oder einer Osteosynthese, ausgebildet sein.

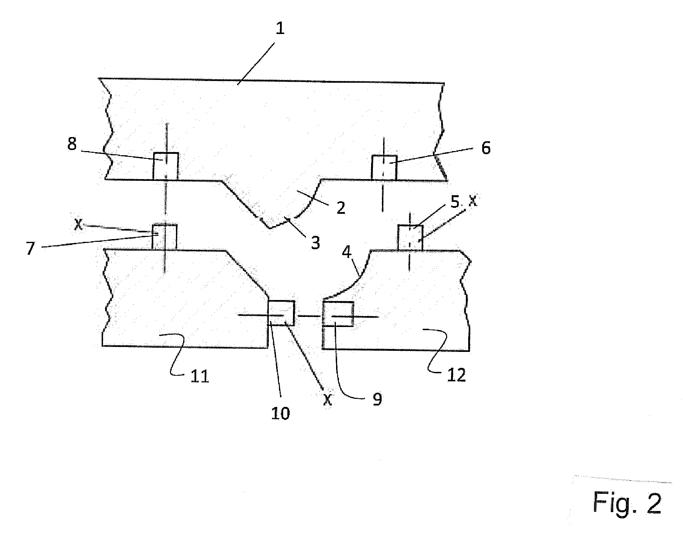

In einer besonderen Ausführungsvariante des Bereitstellens erfolgt das Bereitstellen eines personalisierten Implantat- oder Osteosynthese-Substratkörpers. Derartige Implantat- oder Osteosynthese-Substratkörper und deren Herstellverfahren sind in der EP 16 159 953.5 im Detail beschrieben und werden anhand der Fig. 2-4 näher erläutert.

Der Implantat- oder Osteosynthese-Substratkörper besteht aus einem oder mehreren zusammengesetzten Teilelementen, die vorzugsweise als Kunststoffkörper ausgebildet sind und welche beispielsweise einen Knochen ausbilden.

Der erste Verfahrensschritt I kann u.a. einen 3-D Druck des entsprechenden Implantat- oder Osteosynthese-Substratkörpers umfassen.

Nach der Ausbildung des Implantat- oder Osteosynthese-Substratkörpers kann dieser im Rahmen einer Nachbehandlung nassgefräst werden.

Alternativ oder zusätzlich zu diesem Nassfräsvorgang kann auch ein Abschleifen und/oder Sandstrahlen des Implantat- oder Osteosynthese-Substratkörpers erfolgen.

Ebenfalls alternativ oder zusätzlich kann eine nasschemische Reinigung der Oberfläche des Implantat- oder Osteosynthese-Substratkörpers vorzugsweise mittels einer alkoholischen Lösung, vorzugsweise mit Ethanol und/oder Isopropanol erfolgen. Die nasschemische Reinigung kann bevorzugt in Kombination mit einer Ultraschallreinigung erfolgen.

Das Bereitstellen des Implantat- oder Osteosynthese-Substratkörpers kann zudem eine Oberflächenpolierung umfassen. Diese kann sich bevorzugt an die nasschemische Reinigung anschließen.

In einem zweiten Verfahrensschritt II erfolgt eine physikalische oder physikalisch-chemische Vorbehandlung der Oberfläche des Implantat- oder Osteosynthese-Substratkörpers aus Kunststoff. Dieses Aufbringen erfolgt nach dem sogenannten HiPIMS-Verfahren, deren Auftragung beispielsweise in der WO 2015/038489 A1 im Detail beschrieben wird.

Im vorliegenden Fall kann im Rahmen des Aufbringens der Metallschicht allerdings die Oberflächenhaftung der Metallschicht auf der Kunststoffoberfläche des Implantat- oder Osteosynthese-Substratkörpers durch einige vorbereitenden Schritte weiter verbessert werden.

So kann der Implantat- oder Osteosynthese-Substratkörper zur Verringerung von Materialdefekten einer optionalen Wärmebehandlung bei vorzugsweise 150-250°C für zumindest 1 h, insbesondere für 1,5 bis 2,5 Stunden unterzogen werden.

Im Anschluss an die Wärmebehandlung kann auch ein Ätzvorgang, im Rahmen eines physikalischen oder chemischen Ätzens erfolgen.

Sodann erfolgt eine Plasmaaktivierung der Kunststoffoberfläche des Implantat- oder Osteosynthese-Substratkörpers. Zur Plasmaaktivierung kann der Implantat- oder Osteosynthese-Substratkörper in einen Vakuumsystems überführt werden und sodann kann eine Plasmaaktivierung durch Mikrowellenentladung, induktiv gekoppeltes Plasma (ICP) und/oder RF-Plasmaaktivierung, also Plasmaanregung durch hochfrequente elektrische Felder, erfolgen. Hierzu kann Sauerstoff, insbesondere Reinsauerstoff der Güte 3N, und/oder ein Edelgas eingesetzt werden.

Die Plasmaaktivierung kann vorzugsweise zwischen 3 bis 8 Minuten erfolgen. Die Ionendichte liegt vorzugsweise zwischen 10⁹ und 10¹²/cm² betragen.

Alternativ zur Plasmaaktivierung kann auch eine Ozonierung der Oberfläche des Kunststoffs des Implantats erfolgen.

Somit kann die Aktivierung mit einer Aktivierungsenergie entweder durch Plasmaaktivierung und/oder vorzugsweise durch ein Sauerstoffderivat oder durch eine radikalische Verbindung.

Sobald eine metallische Schicht in einem Verfahrensschritt III abgeschieden wurde, welche für ein HiPIMS-Verfahren hinreichend elektrisch leitend ist, kann ein Wechsel auf von der normalen Sputtermethode auf die HiPIMS-Methode in einem vierten Verfahrensschritt IV erfolgen.

Dabei wird zwischen dem Implantat- oder Osteosynthese-Substratkörper und einer Metallionenquelle ein elektrisches Feld gebildet und positive Metallionen werden von der Metallionenquelle zum Substrat hin beschleunigt.

Dabei wird das HiPIMS-Plasma, anders als bei der herkömmlichen Sputtermethode nicht durch hochfrequente elektrische Felder, also im sogenannten RF-Sputtering erzeugt, sondern es wird durch eine Glimmentladung erzeugt.

Die Stromdichte der Entladung kann vorzugsweise zwischen 2 bis 5 A·cm⁻² betragen, während die Entladungsspannung konstant gehalten wird. Alternativ oder zusätzlich kann die Entladung auch im Leistungskontrollierten Modus betrieben werden mit Peak-Leistungsdichten zwischen 1 und 5 kW/cm². Die erzeugte Plasmadichte beträgt dabei bevorzugt mehr als 10⁹ Ionen pro Kubikzentimer. Die Ionisierung erfolgt durch Elektronenstoß, der durch Ladungsaustausch und Diffusion ausgeglichen wird.

Die mittlere Stromdichte beträgt bevorzugt zwischen 2 und 5 A·cm⁻².

Das Tastverhältnis bei Anwendung der HiPIMS-Methode beträgt zwischen 2 und 20%.

Die am Substrat angelegte negative Spannung, die sogenannte Bias-Spannung, beträgt zumindest 10 V, vorzugsweise 15 bis 100 V.

Durch Synchronisieren (womit Synchronisieren) der Bias-Spannung mit der HiPIMS-Entladung kann ein kontinuierlicher Auftrag von Metallionen auf dem Implantat- oder Osteosynthese-Substratkörper bevorzugt erreicht werden. Ein vermehrter Einbau von Argon in der wachsenden Schicht und damit die Ausbildung von Eigenspannungen kann somit vorteilhaft verhindert werden.

In einem fünften Verfahrensschritt V erfolgt ein Aufbringen einer porösen Schicht. Diese Schicht kann insbesondere durch DC-Sputtern aufgebracht werden, wobei DC als "direct current", also einem Gleichspannungssputtern, zu verstehen ist.

Dabei erfolgt anders als beim HiPIMS-Verfahren kein gepulster Auftrag sondern ein Auftrag von Metallionen mit konstanter Spannung am Target, also dem Implantat- oder Osteosynthese-Substratkörper. Bei diesem DC-Sputtern wird im zeitlichen Verlauf des Auftrags der Metallionen ein Druckgradient angelegt, welcher sich im zeitlichen Verlauf des Auftrages von 5 µbar auf 100 µbar erhöht.

In einem sechsten Verfahrensschritt VI kann eine anodisierte Oberflächenschicht aufgebracht werden oder die Ausbildung einer anodisierten Oberflächenschicht erfolgen. Dies kann durch ein sogenanntes DC-Reaktivsputtern vorzugsweise in einer Sauerstoffatmosphäre erfolgen.

Die aufgebrachte Beschichtung weist somit mehrere Schichten auf und weist eine besonders hohe Anhaftung mit einer Adhäsionsstärke von mehr als 20 MPa, gemäß der Norm ASTM f1147-05 (Stand 2011) (Axial) und ASTM f1044 (Stand 2011) (Scherung), mit einer Standardabweichung von 3MPa auf.

Das Scherverhalten der so beschichteten Kunststoffsubstrate wird durch die Beschichtung nicht verändert.

Durch die Anodisierung der Beschichtung kann eine Farbgebung des Implantat Substratkörpers erfolgen. Dadurch wird dem Operateur bei mehrteiligen Implantatsegmenten im Rahmen einer Operation, beispielsweise einer minimalinversiven Operation, der Zusammenbau des Implantats und/oder der Osteosynthese durch Farbcodierung vereinfacht.

Für die hierin beschriebenen verschiedenen Ausführungsformen wird als Metallionenquelle Titan genutzt, insbesondere Titan nach dem medizinischen Grad 2. besonders bevorzugt Titan mit einer Reinheit von 5 N.

Für den Implantat- oder Osteosynthese-Substratkörper kann vorzugsweise einer oder mehrere Kunststoffe der folgenden Verbindungen genutzt werden: Polyoxymethylen (POM), Polyethylen (PE), Ultrahigh molecular weight Polyethylen (UHMW-PE), Polyetherketon (PEK), insbesondere Polyaryletherketon (PAEK), faserverstärktes PEK, Polyetheretherketon (PEEK), faserverstärkten PEEK, CRF-PEEK, Polyetherketonketon (PEKK), faserverstärktes PEKK, Polyetheretherketonketon (PEEKK), faserverstärktes PEEKK, Polyetherketonetherketonketon (PEKEKK) und/oder faserverstärktes PEKEKK.

### Weitere Materialien können Metalle, Magnesium und/oder Keramiken sein

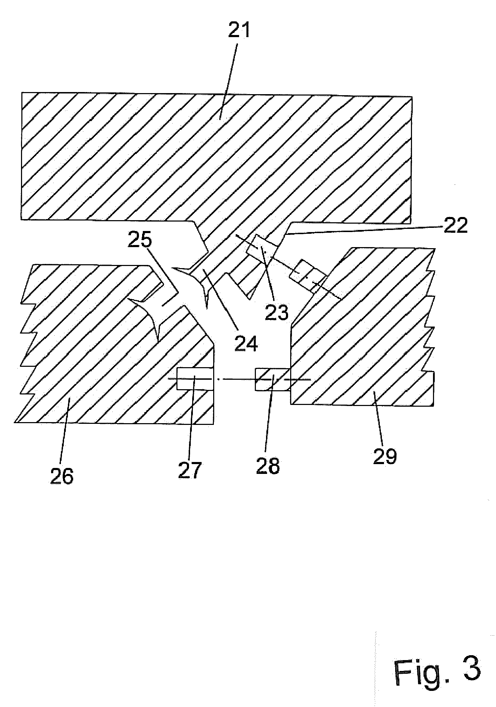

In Fig. 2 und 3 sind zwei unterschiedliche Varianten einer Osteosynthese dargestellt. Je nach Art der mechanischen Belastung an der jeweiligen Position sind die Verbindungsmittel zwischen einzelnen Teilelementen der Osteosynthese gewählt.

Das Bereitstellen eines Implantat- oder Osteosynthese-Substratkörpers kann in einem ersten Verfahrensschritt I derart erfolgen, dass Teilelemente eines mehrteiligen Implantats oder einer mehrteiligen Osteosynthese bereitgestellt werden, welche im Körper zu dem jeweiligen Implantat oder der Osteosynthese zusammengesetzt werden können. Dadurch kann ein chirurgischer Eingriff auch bei einem Einsatz von größeren Implantaten minimalinvasiv und patientenspezifisch erfolgen.

Ein solches bevorzugtes Verfahren zum Bereitstellen von Teilelementen eines mehrteiligen Implantats oder einer mehrteiligen Osteosynthese vor deren Einführung in einen menschlichen und/oder tierischen Körper kann die folgenden Schritte umfassen:
A) Erfassen von Daten eines Patienten, für welchen das Implantat und/oder die Osteosynthese bestimmt ist;
   Als Osteosynthese ist die Gesamtheit aus Verbindungsmitteln einer Verbindung zu verstehen, welche zwischen zwei oder mehr Knochen oder Knochenfragmente eingesetzt werden kann mit dem Ziel, dass diese zusammenwachsen.
   Diese Daten können vorzugsweise Aufnahmen eines bildgebenden Verfahrens sein. Üblicherweise werden diese Aufnahmen von bildgebenden Verfahren zur Diagnose genutzt. Im vorliegenden Fall werden diese Aufnahmen jedoch zur Erstellung eines Modells unabhängig von einer Diagnose erstellt oder alternativ nach erfolgter Diagnose weiterverwandt.
B) Erstellen eines Modells anhand der erfassten Daten;
   Das Erstellen des Modells kann als Computermodell erfolgen. Hierfür kann vorzugsweise ein Computerprogramm genutzt werden, welches auf der Finite-Elemente-Methode beruht. Entsprechende Computerprogramme sind für andere Anwendungen bereits käuflich erhältlich und müssen lediglich für den Anwendungsfall in an sich bekannter Weise umgestellt werden.
C) Erstellen von Fertigungsvorgaben für zumindest zwei oder mehr zu einem Implantat und/oder zu einer Osteosynthese zusammensetzbaren Teilelementen auf Grundlage des erstellten Modells wobei die Fertigungsvorgaben umfassen:
   C1) eine Dimensionierung der Teilelemente
   Die Fertigungsvorgaben werden vorzugsweise anhand des Computermodells derart erstellt, dass die Teilelemente je nach ihrer Funktion unterschiedlich dimensioniert werden. Auch eine Knochenplatte ist an einer Stelle etwas dicker als an einer anderen. Die benötigte Materialdicke und Flexibilität der Teilelemente kann durch das Modell, welches auf patientenspezifischen Daten beruht, individuell ausgestaltet und gefertigt werden.
D) Fertigung der Teilelemente auf Basis der Fertigungsvorgaben; wobei die Teilelemente miteinander zu einem Implantat oder einer Osteosynthese montierbar sind.

Der Schritt der Fertigung von Teilelementen kann auch das Anpassen von vorgefertigten Elementen umfassen, beispielsweise ein Abschleifen einer Vorform anhand einer patientenspezifischen Fertigungsvorgabe.

Losgelöst vom anschließenden chirurgischen Eingriff zum Einführen geht es in dem erfindungsgemäßen Verfahren um das Bereitstellen von Teilelementen, welche zu einem auf den Patienten abgestimmten Implantat oder einer entsprechenden Osteosynthese zusammenfügbar sind.

Durch die Mehrteiligkeit des Implantats oder der Osteosynthese bzw. Verbindung kann der chirurgische Eingriff minimalinvasiv erfolgen und durch die patientenspezifische Anpassung sind der Platzbedarf und die Funktionalität des Implantats besonders optimiert.

### Vorteilhafte Ausgestaltungsvarianten der Erfindung sind Gegenstand der Unteransprüche

Es ist von Vorteil, wenn das Erstellen von Fertigungsvorgaben für zumindest zwei oder mehr zu einem Implantat und/oder zu einer Osteosynthese zusammensetzbaren Teilelementen auf Grundlage des erstellten Modells erfolgt, wobei die Fertigungsvorgaben folgende weitere Angaben umfassen:
C2) eine Materialauswahl bezüglich eines oder mehrerer Materialen für ein jeweiliges Teilelement;
   Dies ermöglicht eine optimale Abstimmung bezüglich einer erwünschten Flexibilität oder Starrheit der Teilelemente.
C3) eine Auswahl einer oder mehrerer Verbindungarten zwischen den jeweiligen Teilelementen.

Dadurch kann eine optimale Verbindung für jedes Teilelement je nach Körperbereich erreicht werden. Dabei kann auf die Verbindung je nach Körperbereich ein hohes Flächengewicht oder erhöhte Zugbelastung wirken. Entsprechend kann die Verbindung gewählt werden.

Diese Angaben verbessern die Anwendungsperformance des Implantats oder der Osteosynthese bzw. der Verbindung.

Die Teilelemente können in vorteilhafter Weise die Teilelemente formschlüssig miteinander verbindbar sein oder zumindest bereichsweise in einem vordefinierten Abstand voneinander beabstandet sind. Ersteres ist besonders bevorzugt um eine feste Anbindung und einen breitflächigen Oberflächenkontakt zu ermöglichen. Der Abstand kann hingegen für eine Verankerung von Gewebe dienen, welche die Verbindung zusätzlich stützt.

Die Fertigungsvorgaben können zudem eine Auswahl von einem oder mehreren Sensoren, Aktuatoren und/oder Wirkstoffen für zumindest eines der Teilelemente umfassen. Dadurch ermöglicht das Implantat eine zusätzliche Überwachung des Gesundheitszustandes des Patienten.

Zum Betrieb und/oder zur Auswertung der Sensorsignale und/oder zur Regelung oder zum Ansteuern der Aktuatoren kann bei der Fertigung der Teilelemente eine Integration und/oder Fixierung einer Stromversorgungsquelle und/oder einer Steuer- und/oder Auswerteeinheit an oder in einem der Teilelemente erfolgen.

Es kann zudem vorteilhaft bei der Fertigung einer Sendeeinheit an oder in dem Implantat oder der Osteosynthese angeordnet werden. Die Sendeeinheit kann eine aktive oder passive Sendeeinheit sein.

Das Erstellen des Modells kann vorteilhaft durch Erstellen eines dreidimensionalen Computer-Modells, vorzugsweise nach der Finite-Elemente-Methode, erfolgen.

Das Erfassen von Daten kann vorteilhaft durch ein bildgebendes Verfahren zum Zwecke des Erstellens eines dreidimensionalen Modells erfolgen.

Anders als im Stand der Technik sind die Teilelemente zumindest im Bereich der Außenkontur des Implantats oder der Osteosynthese individuell angepasst. Daher sind vorteilhaft zumindest 66% aller Teilelemente unterschiedlich zueinander dimensioniert gefertigt sind und besonders bevorzugt zumindest 80% aller Teilelemente unterschiedlich zueinander dimensioniert gefertigt sind. Beim Stand der Technik sind bei mehrteiligen Implantaten zumeist nur zwei Typen von Teilelementen vorgesehen, also sind 50% aller Teilelemente identisch dimensioniert. Dies ermöglicht eine besonders kleinteilige und individualisierte Anpassung.

Die Teilelemente im zusammengesetzten Zustand eines Implantats und/oder einer Osteosynthese definieren vorteilhaft ein Kantenmodell mit einem größeren Volumen als jedes einzelne Teilelement, vorzugsweise ein dreifach größeres Kantenmodell, insbesondere ein 8-fach größeres Kantenmodell.

Die Teilelemente weisen vorzugsweise aufgrund ihrer unterschiedlichen Funktionalität unterschiedliche Materialien auf. So ist in einer vorteilhaften Ausführungsvariante ein erstes Teilelement der Teilelemente aus einem ersten Material und/oder einer ersten Materialkombination gefertigt und ein zweites Teilelement der Teilelemente aus einem zweiten Material und/oder einer zweiten Materialkombination gefertigt. Eine erste Materialkombination kann z.B. Gummi und Titan als Verbundelement sein und eine zweite Materialkombination PLA und Titan oder auch nur reines Titan.

Die auf das Volumen gemittelte Dichte und/oder das Volumen gemittelte Elastizitätsmodul des ersten Materials und/oder der ersten Materialkombination ist dabei größer als die auf das Volumen gemittelte Dichte und/oder das auf das Volumen gemittelte Elastizitätsmodul der zweiten Materialkombination. Das heißt man ermittelt die Dichte und/oder das E-Modul des Gummis und multipliziert diesen Wert mit dem prozentualen Volumenanteil dieses Materials im Teilelement. Sodann ermittelt man die Dichte und/oder das E-Modul des Titans und multipliziert diesen Wert mit dem prozentualen Volumenanteil des Titans im Teilelement. Die beiden Werte werden dann addiert und auf hundert Prozent normiert und können dann mit dem entsprechenden Wert des Materials oder der Materialkombination des zweiten Teilelements verglichen werden. Die erste und/oder zweite Materialkombination braucht sich dabei auch nur hinsichtlich des Anteils der Einzelkomponenten in der Materialkombination zu unterscheiden, z.B. hinsichtlich des Anteils an Gummi oder Titan. Das E-Modul und/oder die Dichte und/oder das Volumen der Materialkomponenten kann unter Standardbedingungen mit im Fachgebiet üblichen Messgeräten unter Berücksichtigung der üblichen Schwankungsbreiten ermittelt werden. Der Nachweis des Volumens kann durch Abmessung, ggf. unter Zuhilfenahme einer laserunterstützten Scanmethode, bestimmt werden. Die Dichte der Implantate oder einzelner Teilelemente kann durch radiologische Bestimmung z.B. mittels CT, beispielsweise durch das Gerät Somatom Flash von Siemens ermittelt werden. Die Elastizität der Teilelemente des Implantats oder der Osteosynthese, insbesondere auch der Gewebeanteile kann mittels des Messgeräts Bioindenter an der CSEM nachgewiesen werden. Durch die unterschiedlichen Dichten und E-Module können die Teilelemente sehr individuell auf die Situation des Patienten angepasst werden.

Dabei ist es insbesondere von Vorteil, wenn ein erstes Teilelement der Teilelemente aus einem ersten Material und/oder einer ersten Materialkombination, z.B. einem Verbundmaterial, gefertigt wird und ein zweites Teilelement der Teilelemente aus einem zweiten Material und/oder einer zweiten Materialkombination gefertigt wird, wobei die auf das Volumen gemittelte Dichte und/oder das Volumen gemittelte Elastizitätsmodul des ersten Materials und/oder der ersten Materialkombination größer ist, vorzugsweise zumindest 1,2-fach größer ist, besonders bevorzugt zumindest 1,5-fach größer ist als die auf das Volumen gemittelte Dichte und/oder das auf das Volumen gemittelte Elastizitätsmodul des zweiten Materials und/oder der zweiten Materialkombination.

Alternativ oder zusätzlich kann ein Teilelement aus einem ersten Material und aus einem zweiten Material gefertigt sein, wobei die Dichte und/oder das Elastizitätsmodul des ersten Materials vorteilhaft größer ist, vorzugsweise zumindest 1,2-fach größer ist, besonders bevorzugt zumindest 1,5-fach größer ist als die Dichte und/oder das Elastizitätsmodul des zweiten Materials.

Der oder die Aktuatoren und/oder der oder die Sensoren eines oder mehrerer Teilelemente sind vorzugsweise und vorteilhaft in MEMS-Bauweise ausgebildet und die Sensorelemente und/oder Aktuatorelemente, ohne Auswerteeinheit und/oder Stromversorgungseinheit, sind besonders bevorzugt kleiner als 1 mm³.

Eines oder mehrere Teilelemente können vorteilhaft aus einem resorbierbaren Material und besonders bevorzugt aus einem resorbierbaren Kunststoff oder Magnesium bestehen. Diese ermöglichen einen unterstützenden, jedoch nicht dauerhaften Aufbau von Körperteilen.

Für eine einfache Montage ist es von Vorteil, wenn die Verbindungen der Teilelemente als Steckverbindungen ausgestaltet sind. Diese Steckverbindungen können besonders bevorzugt über einen zusätzlichen Einrast-Mechanismus verfügen, welche die Teilelemente nach dem Zusammenstecken miteinander verrastet. Alternativ und ebenfalls vorteilhaft können die Teilelemente über ein Filmscharnier miteinander verbunden sein oder eine Klebung.

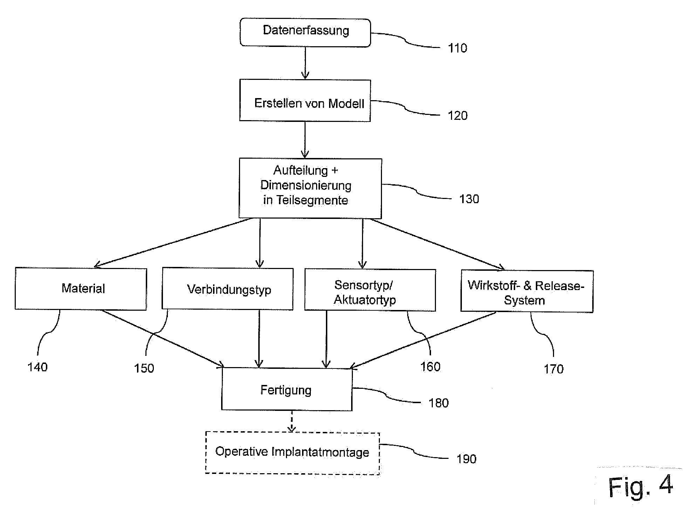

Fig. 4 zeigt einen Verfahrensablauf eines erfindungsgemäßen Verfahrens zur Bereitstellung eines mehrteiligen Implantats oder einer mehrteiligen Osteosynthese.

In einem ersten Schritt erfolgt eine Datenerfassung 110. Bei dieser Datenerfassung handelt es sich um eine individuelle Datenerfassung eines Patienten. Es handelt sich somit um individuelle Patientendaten, welche vorzugsweise auf Basis von Bildgebungsverfahren bzw. bildgebende Verfahren erstellt wurden.

Derlei Verfahren ermöglichen die Aufnahmen von Organen und/oder Knochen, auf deren Basis anschließend ein Modell erstellt werden kann. Bildgebende Verfahren basieren bevorzugt auf folgenden Signalen, aus welchen im Anschluss ein Bild einzelner Organe, Knochen oder ähnliches für den individuellen Patienten erstellt werden können:
- Kernspinresonanz, z. B. MRT-Aufnahmen
- Ultraschall, z. B. sonographische Aufnahmen
- Röntgenstrahlungen, z. B. Röntgenaufnahmen,
- Inforarotstrahlung z. B. thermographische Aufnahmen,
- Licht im VIS-Bereich, z. B. Endoskopische Aufnahmen
- Impedanz, z. B. EIT-Aufnahmen
- Radionuklide z. B. szintigraphische Aufnahmen

Besonders bevorzugt sind dreidimensionale Aufnahmen von bildgebenden Verfahren und somit sind bildgebende Verfahren zur Datenerfassung bevorzugt, welche dreidimensionale Aufnahmen ermöglichen.

Zusätzlich zur Erfassung von dreidimensionalen Aufnahmen, kann auch ein Erfassen weiterer chemischer und/oder physikalischer Daten erfolgen. Dies kann beispielsweise das Erfassen der Konzentration spezifischer Verbindungen im Körper, z.B. einer Ionenkonzentration und/oder des pH-Werts, der Körpertemperatur, des Herzschlags und/oder des Kontraktions- und Expansionsvolumens einzelner Organe umfassen. Diese Werte sind teilweise ebenfalls patientenspezifisch.

Auf Basis dieser Datenerfassung kann ein Modell 120 eines betroffenen Organs und/oder eines betroffenen Knochens und/oder der Umgebung des Organs und/oder Knochens erstellt werden. Handelt es sich bei dem Modell um die Umgebung, so kann aufgrund dieses Modells ein Raumbedarf des benötigten Implantats und/oder der benötigten Osteosynthese ermittelt werden.

Bei dem Modell 120 kann es sich um ein digitales dreidimensionales ComputerModell handeln oder um ein reales dreidimensionales Computermodell, welches vorzugsweise durch Stereolithographie, besonders bevorzugt durch Rapid-Prototyping, und insbesondere durch ein generatives Fertigungsverfahren, wie z.B. selektives Laserschmelzen oder durch einen 3D-Druck hergestellt wurde.

Anhand dieses Modells 120 werden vor der Ausgestaltung des Implantats und/oder der Osteosynthese Fertigungsvorgaben erstellt, dies umfasst u. a. eine Aufteilung und Dimensionierung 130 von Einzelteilen bzw. Teilelementen des dreidimensionalen individualisierten Implantats oder der dreidimensionalen individualisierten Osteosynthese.

Das Modell 120 und die Teilelemente des Modells können zur Definition ihrer dreidimensionalen Ausbreitung jeweils durch ein imaginäres quaderförmiges Kantenmodell begrenzt werden, wobei diese Kantenmodelle die maximale Ausdehnung des Modells 120 und die maximale Ausdehnung der Teilelemente des Modells aufweisen. Somit ist jedes der Teilelemente kleiner als das Gesamtkonstrukt des Implantats und/oder der Osteosynthese.

Dabei ist das Volumen des Kantenmodells, welches das Modell 120 des Implantats oder der Osteosynthese begrenzt, größer als das Volumen des Kantenmodells, welches ein Teilelement des Modells 120 begrenzt.

Vorzugsweise ist das Volumen des Kantenmodells des Modells 120 zumindest dreimal größer, besonders bevorzugt zumindest 8-fach größer, als das Volumen des Kantenmodells des Teilelements des Modells 120.

Besonders bevorzugt weist zumindest die Querschnittsfläche eines Teilelements einen geringeren Flächeninhalt auf als die Querschnittsfläche des Implantats oder der Osteosynthese welche die Querschnittsfläche des Teilelements beinhaltet. Das heißt der Querschnitt des Implantats oder der Osteosynthese auf Höhe des Querschnitts des Teilelements ist größer als der des Teilelements, vorzugsweise zumindest doppelt so groß, besonders bevorzugt zumindest 4-mal so groß.

Die Definition gemäß Kantenmodell bezieht sich dabei auf ein Modell des Implantats und/oder der Osteosynthese. Handelt es sich bei dem Modell um ein Modell der Umgebung, so ist anhand des Modells der dreidimensionale Raumbedarf des benötigten Implantats oder der benötigten Osteosynthese zu erstellen und das Kantenmodell ist auf Grundlage dieses Raumbedarfs zu erstellen.

Durch das Modell 120 wird das Makrodesign des bereitzustellenden Implantats oder der bereitzustellenden Osteosynthese abgestimmt, welches Implantat und/oder welche Osteosynthese somit individuell und optimal in allen drei Raumrichtungen auf den Patienten angepasst sind. Dabei ändern sich typischerweise die Abmessungen des Modells und des nach dem Vorgaben des Modells modellierten Implantats oder der Osteosynthese für jeden Patienten in allen drei Raumrichtungen.

Nach der Aufteilung des Modells in Teilelemente 130 erfolgt eine segmentspezifische Auswahl zur Ausgestaltung hinsichtlich eines jeweiligen Teilelements.

Diese Auswahl betrifft u. a. die Auswahl des Materials 140 eines jeweiligen Teilelements und die Auswahl der Verbindung 150 der Teilelemente untereinander.

Die Materialauswahl 140 kann besonders bevorzugt eine oder mehrere der folgenden Materialien umfassen: Formgedächtnis-Material, Self-Assembly-Material, Gradienten-Material, künstliche Muskeln und/oder Gewebeanteile, vorzugsweise Haut, Schleimhaut, Knochenhaut und/oder Weichgewebe.

Formgedächtnismaterialien können vorzugsweise aus Kunststoff oder einer Legierung, einem sogenannten shape-memory-alloy, bestehen.

Selbstassemblierung (Self-Assembly-Materialien) sind beispielsweise Materialien, die autonom, also ohne äußerliche Einwirkungen eine spezifische Struktur- und Musterbildung zeigen. Zudem können sie aber auch unter Anregung, wie z.B. Licht, mechanische Anregung oder elektrische Anregung, eine neue Form annehmen. Dies kann z.B. eine Struktur mit starren Teilbereichen sein, welche mit Filmscharnieren verbunden ist und welche minimal-invasiv in eine Wunde einführbar und anschließend entfaltbar ist. Das Entfalten kann bevorzugt zu einer vordefinierten Form erfolgen.

Gradienten-Materialien werden aktuell vom Fraunhofer Institut untersucht und entwickelt. Ein Gradienten-Material ist beispielsweise PCU, Polycarbonaturethan.

Künstliche Muskeln werden derzeit u.a. unter dem Projekt "Bionicum" in verschiedenen Forschungsinstituten untersucht. Derartige Muskeln kommen für die Materialauswahl der Teilelemente ebenfalls in Betracht.

Weiterhin ist auch bekannt Gewebe, insbesondere menschliches Gewebe, zu züchten. Dieses Gewebe kann ebenfalls als Material für die Teilelemente eingesetzt werden. Zu dem Gewebe sind u.a. Haut, Schleimhaut, Knochenhaut und Weichgewebe gezählt werden. Alternativ zur Züchtung kann das Gewebe auch dem Patienten oder einem Spender, menschlich oder tierisch, entnommen werden und bei der Materialauswahl berücksichtigt werden.

Darüber hinaus sind weitere bevorzugte Materialien für die Materialauswahl Materialien mit Fest-Flüssig oder Fest-Fest - Phasenumwandlungen unter Wärmeeinfluss im Temperaturbereich zwischen 0 bis 85°C oder Lichteinfluss, Graphen-Materialien, magnetische Materialien, Materialkombinationen mit unterschiedlicher Röntgenopazität, thermisch-leitende oder elektrisch-leitende Materialien.

Bei der Materialauswahl des Basismaterials können sowohl Metalle/Legierungen als auch keramische Materialien und/oder Kunststoffe genutzt werden, welche zumindest teilweise mit einer Titanschicht gemäß dem erfindungsgemäßen Verfahren versehen werden. Es können auch für einige Teilelementen massives Titan genutzt werden und für einige Teilelemente des Implantats oder der Osteosynthese die vorgenannten Materialien, welche erfindungsgemäß mit einer Titanschicht versehen sind. In einer bevorzugten Ausführungsvariante der Erfindung können insbesondere faserverstärkte Kunststoffe für die Herstellung der Teilelemente genutzt werden, wie z.B. karbonverstärktes Polyetheretherketon (PEEK). Es sind auch Materialkombinationen und Materialverbundstoffe möglich, welche in einem Teilelement vorkommen können. Je nach Beanspruchung des Implantats und/oder der Osteosynthese kann es darauf ankommen, dass ein erstes Teilelement besonders flexibel ausgebildet ist und ein zweites Teilelement des gleichen Implantats oder der gleichen Osteosynthese besonders flexibel, also biegsam und/oder dehnbar und/oder schaumartig, ist. Dies kann durch die Materialauswahl vorab bestimmt und angepasst sein. Jeder Patient hat dabei andere Körpermaße und muskuläre Ausprägungen, sodass die Dimensionierung des Implantats oder der Osteosynthese anhand des vorher erstellten Modells individuell vorgenommen werden kann.

Zu den bevorzugten keramischen Werkstoffen zählen insbesondere auch Werkstoffe aus Magnesium.

Besonders bevorzugt verwendete Kunststoffe sind resorbierbare Kunststoffe vorzugsweise basierend auf Polymilchsäure und/oder deren Derivate, z.B. PLA. Die Kunststoffe können bevorzugt zumindest bereichsweise faserverstärkt sein.

Gleiches gilt für die Auswahl des Typs der Verbindungen zwischen den Teilelementen. Die Verbindung kann teilweise flexibel sein. Dies kann über Kugelgelenke erreicht werden, wie sie z.B. in der US 6 060 641 A offenbart sind. Sie kann allerdings auch starr sein. Verschiedene Verbindungstypen sind in den Fig. 1-4 näher dargestellt. Die vorliegende Erfindung unterscheidet zwischen Verbindungsmitteln, welche Teilelemente des Implantats oder der Osteosynthese miteinander verbinden und zwischen Fixationsmitteln welche das Implantat oder die Osteosynthese an einer bestehenden Struktur z. B. einem Knochengerüst im Körper fixieren. Die Verbindungsmittel können allerdings zugleich auch als Fixationsmittel dienen

Optional kann auch eine Auswahl hinsichtlich von Sensoren und/oder Aktuatoren 160 erfolgen, welche in dem jeweiligen Teilelement integriert sein können. Sensoren können beispielsweise die Körpertemperatur oder den Herzschlag erfassen und an einen Aktuator in Form eines Herzschrittmachers weitergeben, welcher elektrische Impulse zur Stimulation des Herzmuskels aussendet.

Aktuatoren sind dabei Elemente welche elektrische Signale in mechanische oder andere physikalische Größen, z. B. Wärme oder Licht, umsetzen. Dies kann z.B. ein Heizelement sein, welches die Temperatur in einem spezifischen Bereich des Körpers reguliert. Auch Chemostate sind im Rahmen der vorliegenden Erfindung als Aktuatoren zu verstehen. Sie dienen vorzugsweise zur Regulierung eines pH-Wertes, einer Ionen- und/oder Sauerstoffkonzentration. Die Aktuatoren können in mikromechanischer bzw. mikroelektromechanischer Bauweise ausgeführt sein.

Sensoren, welche im Rahmen der vorliegenden Erfindung in Teilelementen von Implantaten und/oder Osteosynthesen eingesetzt werden können, können z. B. chemische, elektrische, elektrochemische und/oder physikalische Sensoren zum Erfassen einer patientenspezifischen Größe sein. Bevorzugt eingesetzte Sensoren sind unter dem Begriff "BioMEMS" bekannt. Dabei handelt es sich um mikroelektromechanische Sensoren für den Anwendungsbereich der Diagnostik und der Biotechnologie. Sie umfassen vorzugsweise elektrochemische Sensoren.

Entsprechende Sensoren in mikromechanischer Bauweise als MEMS-Elemente, welche über Festphasen-Elektroden als Referenzelektrode verfügen, können in einem Teilelement eines Implantats oder einer Osteosynthese integriert werden.

Es sind auch Durchflusssensoren bekannt, welche als mikroelektromechanische Sensoren ausgebildet sind. Diese können ebenfalls beispielsweise zur Überwachung einer lokalen Blutzirkulation in einem Teilsegment des Implantat und/oder der Osteosynthese integriert sein.

Es sind zudem Optische Sensoren, Piezoresistive Sensoren, Drucksensoren und/oder Temperatursensoren bekannt, welche vorzugsweise in mikromechanischer Bauweise ausgeführt sein können und welche im Rahmen der vorliegenden Erfindung in einem Teilelement eines Implantats und/oder einer Osteosynthese integriert sein können.

Weiterhin kann das Implantat oder die Osteosynthese auch eine Stromversorgungsquelle aufweisen zur Energieversorgung der vorgenannten Aktuatoren und/oder Sensoren.

Weiterhin kann das Implantat oder die Osteosynthese eine Auswerteeinheit aufweisen, welche die von einem oder mehreren Sensoren ermittelten Daten erfasst und ggf. auswertet oder an eine externe Recheneinheit, also eine Recheneinheit außerhalb des Körpers, zur weiteren Auswertung, vorzugsweise durch Wireless-Datenübertragung, übermittelt. Zudem kann die Steuereinheit aufgrund der ermittelten Daten einen oder mehrere der vorgenannten Aktuatoren ansteuern. In einer bevorzugten Variante kann die Steuer- und die Auswerteeinheit als ein Bauteil realisiert sein. Die Steuerung kann sowohl digital als auch analog erfolgen und kann sowohl ein Zusammenwirken von Teilelementen erreichen als auch eine Kommunikation mit Geräten außerhalb des Körpers.

Weiterhin optional kann eine Auswahl von Wirkstoff- und/oder Releasesystemen 170 erfolgen, mit welchen ein Teilelement des Implantats oder der Osteosynthese beschichtet wird oder welche im Material des Teilelements integriert bzw. eingebunden werden.

Unter dem Begriff Wirkstoffe bzw. Wirkstoffsysteme sind Arzneistoffe zu verstehen. Dies können z.B. Antibiotika, Immunsuppressiva, Wachstumshormone oder Zytostatika sein.

Releasesysteme umfassen alle Substanzen, welche typischerweise zusätzlich dem Arzneistoff zugegeben werden, beispielsweise um die Freisetzung des Arzneistoffes im Körper hinsichtlich des Abgabezeitraumes und des Abgabeortes zu steuern oder um den Arzneistoff unter verschiedenen Bedingungen haltbar zu machen. Typische Substanzen sind z.B. Tocopherol, welches oft als Antioxidans zugesetzt wird, oder Polysaccharide, z.B. Chitin, zur Mikroverkapselung eines entsprechenden Arzneistoffes. Die Wahl des Releasesystems hängt u. a. auch von der Wahl des Materials, also des Basismaterials, ab aus welchem das Teilelement hergestellt werden soll.

In einem weiteren Schritt erfolgt die Fertigung 180 der Teilelemente entsprechend der vorgenannten Auswahlkriterien. Die jeweiligen Teilelemente können vorzugsweise durch generative Fertigungsverfahren, z. B. SLM-Verfahren, durch 3D-Druckverfahren und/oder durch mechanische, insbesondere materialabtragende Verfahren, wie z. B. Fräsen, anhand des Modells ausgebildet werden. Anschließend wird im Rahmen der Fertigung 180 die Beschichtung nach dem erfindungsgemäßen Verfahren aufgetragen. Dies kann z.B. nach der Verfahrensvariante der Fig. 1 erfolgen.

Die Teilelemente können durch eine operative Montage 190 zu einem Implantat oder einer Osteosynthese zusammengesetzt werden. Dabei können die Teilelemente des Implantats oder der Osteosynthese im Rahmen eines operativen Eingriffs durch eine Operationswunde in den menschlichen Körper eingeführt und im Körper zum Implantat oder zur Osteosynthese zusammengesetzt bzw. montiert werden. Die Montage kann zudem die Fixierung des Implantats z. B. an einem bestehenden Knochengerüst umfassen.

Das Einführen der Teilelemente des Implantats oder der Osteosynthese ermöglicht eine Verringerung der Dimensionierung einer Operationswunde, sodass das Einsetzen des individuell auf den Patient angepassten Implantats oder der Osteosynthese im Rahmen einer minimalinvasiven Vorgehensweise bei einer Operation erfolgen kann, wodurch die Chancen und der Zeitraum einer erfolgreichen Verheilung und der Regeneration deutlich erhöht wird.

Der operative Eingriff umfassend das Einführen der Teilelemente durch die Operationswunde und deren Zusammensetzung zu einem Implantat oder einer Osteosynthese kann endoskopisch, von Hand, navigiert und/oder roboter-unterstützt erfolgen.

In Fig. 2 und 3 sind ausschnittsweise einzelne Beispiele für Verbindungen der Teilelemente zu einem Implantat oder einer Osteosynthese dargestellt. Diese Verbindung erfolgt im Körper vorzugsweise im Rahmen einer minimalinvasiven Operation.

In Fig. 2 erkennt man die Teilelemente 1, und 12. Dabei kann es sich beispielsweise um Knochenplatten aus PLA-Kunststoff handeln. Diese weisen ungleichmäßige Strukturen mit Vorsprüngen 2 und Aufnahmen 4 auf, welche auf, deren Kontaktflächen 3 unter einen Formenschluss der Teilelemente 1, 11 und 12 aneinander anliegen.

Die Form der Teilelemente und deren Material können bevorzugt aufgrund einer simulierten Belastungsanalyse erfolgen. Entsprechende Simulationsprogramme sind kommerziell erhältlich und können z.B. als CAD-System im Rahmen der Finiten Elemente-Methode ermittelt werden.

Die Teilelemente 1, 11 und 12 weisen zudem gleichartige Vorsprünge 5, 7, 10 und Aufnahmen 6, 8, 9 auf um eine kraft- und formschlüssige Verbindung in der bevorzugten Form einer Steckverbindung vorzugsweise in mehrere Raumrichtungen zu ermöglichen.

Als Verbindungen zwischen Teilelementen ist auch eine oder mehrere bionische Verbindungen bevorzugt. Diese bionische Verbindung kann vorzugsweise durch körpereigenes Gewebe, z.B. Muskeln, angesteuert werden oder durch Nerventransmission.

Fig. 3 zeigt unterschiedliche Arten von Steckverbindungen, deren Vorsprünge und Aufnahme an Teilelementen 21, 26, 29 angeordnet sind. Teilelement 29 weist gleichartige Vorsprünge 28 auf, welche in unterschiedlichen Raumrichtungen aus dem Teilelement hervorstehen. Teilelement 26 weist zwei Aufnahmen 25 und 27 auf, welche unterschiedliche geometrische Ausgestaltungen aufweisen. Während eine erste Aufnahme 25 zylindrisch ausgebildet ist, weist die zweite der Aufnahmen 25 einen Hinterschnitt auf. In diese Aufnahme mit Hinterschnitt greift formschlüssig und kraftschlüssig ein Vorsprung 24 des Teilelements 21 ein. Dieses Teilelement 21 weist zudem eine Aufnahme 3 auf in welche der Vorsprung 28 form- und kraftschlüssig einsteckbar ist.

Die Fig. 2-3 stellen nur einige bevorzugte Beispiele für Verbindungen dar. Es sind jedoch auch Bajonettverbindungen oder andere Verbindungsvarianten der Teilelemente im Rahmen der vorliegenden Erfindung realisierbar

In einer bevorzugten Ausführungsvariante der Erfindung kann ein Teilelement oder eine Verbindung eine Sollbruchstelle aufweisen, in welchem das montierte Implantat oder die montierte Osteosynthese bei stärkerer mechanischer Belastung bevorzugt bricht.

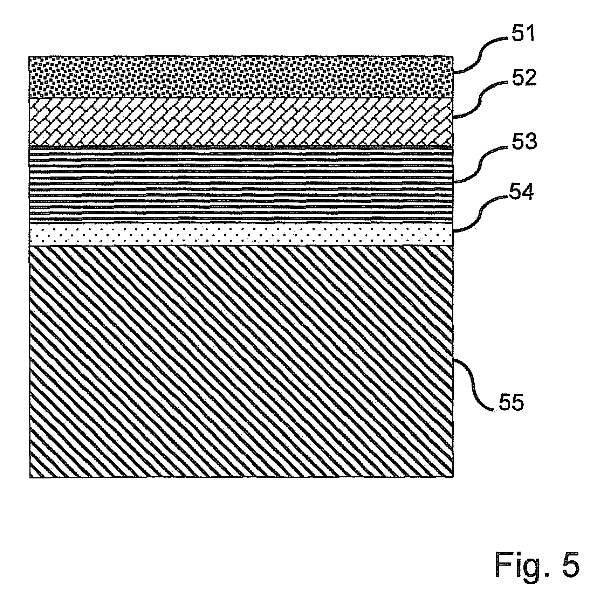

Fig. 5 zeigt eine Detailansicht eines Schnittes der Oberfläche der Beschichtung 50 eines erfindungsgemäßen Implantats oder einer erfindungsgemäßen Osteosynthese. Sowohl beim Implantat als auch bei der Osteosynthese handelt es sich um chirurgische Elemente zum Einsatz in den menschlichen und/oder tierischen Körper.

Eine zur Umgebung hin oberste erste Schicht 51 ist eine durch Anodisierung hergestellte Schicht, welche zu mehr als 80 Gew.% Titanoxid und/oder Titanhydroxyapatit umfasst. Diese Schicht wird in Wesentlichen durch das Anodisieren im sechsten Verfahrensschritt VI ausgebildet.

Der Zweck dieser Schicht ist eine poröse Struktur bereitzustellen, da die Anodisierung durch Einbau von Sauerstoff eine massive Volumenzunahme bewirkt. Durch die poröse Struktur werden Eigenspannungen vermieden, die bei der Anodisierung von dichten HiPIMS-abgeschiedenen Schichten entstünden.

Somit wird gegenüber dem in der WO2015038489 A1 beschriebenen Implantat, welches nach dem HiPIMS-Verfahren mit Titan beschichtet wurde, ein deutlich besseres Implantat geschaffen, welches eine zusätzliche durch Anodisierung aufgebrachte Schicht 51 mit einer metalloxid- und/oder metallhydroxyapatithaltigen durch Sputtern aufgebrachten Schicht 52 aufweist.

Unterhalb der vorgenannten obersten Schicht 51 und besonders bevorzugt benachbart zu dieser weist das erfindungsgemäße Implantat oder die erfindungsgemäße Osteosynthese eine zweite Schicht 52 auf, welche zu mehr als 80 Gew.% poröses elementares Titan umfasst. Dabei weist diese zweite Schicht 52 eine Dichte auf, welche zwischen 50 bis 95% der theoretischen Dichte entspricht. Die Dichte kann z.B. mittels Rutherford Backscatter Spectroscopy (RBS) ermittelt werden. Die Dicke dieser Schicht beträgt vorzugsweise zwischen 200 nm und 2 µm.

Die poröse Titanschicht kann nachträglich noch oxidiert sein. In diesem Fall umfasst die zweite Schicht 52 zumindest zu 20 Gew.% Titanoxid.

Bei einer Volumenausdehnung z.B. durch Wärmezufuhr kann es zwischen der obersten ersten Schicht 51 gegenüber der dritten Schicht 53 zu Materialspannungen kommen und zu einem teilweisen Ablösen der ersten Schicht 51 kommen. Diese Materialspannungen können jedoch durch die zweite poröse Schicht ausgeglichen werden.

Unterhalb der zweiten Schicht 52 und besonders bevorzugt benachbart zu dieser weist das erfindungsgemäße Implantat oder die erfindungsgemäße Osteosynthese eine dritte Schicht 53 auf, welche zu mehr als 95 Gew.% elementares Titan umfasst. Die Dichte dieser dritten Schicht 53 beträgt zumindest 95 % der theoretischen Dichte. Die bevorzugte Schichtdicke dieser dritten Schicht beträgt5 zwischen 50 bis 300 nm.

Unterhalb der vorgenannten dritten Schicht 53 und besonders bevorzugt benachbart zu dieser weist das erfindungsgemäße Implantat oder die erfindungsgemäße Osteosynthese eine vierte Schicht 54 auf, welche nachfolgend auch als Implantationszone bezeichnet wird. Diese Schicht bzw. dieser Bereich umfasst das Material des Implantat- und/oder des Osteosynthese-Substratkörpers, z.B. Kunststoffmaterial, und darin dispergierte Metallatome, insbesondere Titanatome.

Schließlich folgt der Implantat- und/oder des Osteosynthese-Substratkörper 55 auf die vierte Schicht 54.

Das Material des Implantat- und/oder des Osteosynthese-Substratkörpers sollte bevorzugt ein Kunststoffmaterial sein mit einer Temperaturstabilität bis zu 120°C. Bis zu dieser Temperatur sollte sich das Material nicht chemisch zersetzen oder im starken Maße verformen.

Die HiPIMS-Beschichtung zeichnet sich durch eine geringe mittlere Schichtdicke von maximal 2 µm aus. Die untere Grenze einer mittleren Schichtdicke liegt bei 10 nm. Besonders bevorzugt liegt die mittlere Schichtdicke der Beschichtung zwischen 200 bis 500 nm.

Dies ist besonders von Vorteil, da die geringe Beschichtungsstärke eine Reihe von bildgebende Techniken, so z.B. MRI oder Röntgenstrahlen, nicht stört.

Das erfindungsgemäße Beschichtungsverfahren ist insbesondere ein substratkonformer Auftrag von Titan. So kommt es zu keiner Veränderung der Oberfläche des Kunststoffes während des Metallauftrags, insbesondere wird die Oberfläche des Kunststoffs des Implantat- oder Osteosynthese-Substratkörpers bei der metallischen Abscheidung nicht aufgeschmolzen.

Die Übergangszone bzw. das Interface zwischen der mehrschichtig aufgebauten Metallbeschichtung und dem Implantat- oder Osteosynthese-Substratkörpers aus Kunststoff beträgt vorzugsweise weniger als 100 nm, insbesondere unter 10 nm.

Darüber hinaus ergibt sich für die nach dem erfindungsgemäßen Verfahren aufgebrachte Metallschicht eine besonders gleichmäßige Beschichtungsverteilung u.a. auch für die Beschichtung in Kavitäten. Aus diesem Grunde eignet sich das Verfahren besonders zur Herstellung von titanbeschichteten Implantaten und/oder Osteosynthesen.

So ergibt sich für einen 1 mm breiten und 2 cm tiefen Riss im Implantat eine Beschichtungsstärke von zumindest 30%, vorzugsweise von 50% bezogen auf die Schichtdicke einer Beschichtung auf einer flachen Oberfläche ohne Riss.

Die metallische Oberfläche des Implantats und/oder der Osteosynthese kann vorzugsweise superhydriphil ausgebildet sein. Dies kann beispielsweise durch eine optionale Plasmabehandlung der metallischen Oberfläche mit Radikalen erfolgen.

Einzelne Schichten der Fig. 5 oder auch die gesamte metallische Beschichtung können durch Maskieren nur bereichsweise auf dem erfindungsgemäßen Implantat und/oder der erfindungsgemäßen Osteosynthese ausgebildet sein.

Alternativ zu Titan kann auch Tantal als Beschichtungsmaterial genutzt werden.

### Bezugszeichenliste

- Verfahrensschritt I: Bereitstellen des Substratkörpers
- Verfahrensschritt II: Vorbehandlung
- Verfahrensschritt III: Auftrag einer elektrisch-leitenden Schicht
- Verfahrensschritt IV: HiPIMS Sputtern
- Verfahrensschritt V: DC Sputtern
- Verfahrensschritt VI: DC Reaktivsputtern

- 1: Teilelement
- 2: Vorsprung
- 3: Aufnahme
- 4: Kontaktfläche
- 5: Vorsprung
- 6: Aufnahme
- 7: Vorsprung
- 8: Aufnahme
- 9: Aufnahme
- 10: Vorsprung
- 11: Teilelement
- 12: Teilelement

- 21: Teilelement
- 22: Kontaktfläche
- 23: Aufnahme
- 24: Vorsprung
- 25: Aufnahme
- 26: Teilelement
- 27: Aufnahme
- 28: Vorsprünge
- 29: Teilelement

- 110: Datenerfassung
- 120: Erstellen von Modell
- 130: Aufteilung + Dimensionierung in Teilelemente
- 140: Material
- 150: Verbindungstyp
- 160: Sensortyp/Aktuatortyp
- 170: Wirkstoff- & Release-System
- 180: Fertigung
- 190: Operative Implantatmontage

## Patentansprüche

1. Verfahren zur Herstellung eines Implantats oder einer Osteosynthese mit einem nichtmetallischen Substratkörper (55) und einer darauf zumindest bereichsweise angeordneten mehrschichtigen Beschichtung (51-54) umfassend eine metallische Schicht (53) und eine metalloxid- und/oder metallhydroxyapatithaltige Schicht (51), **gekennzeichnet durch** die folgenden Schritte:
a. Bereitstellen des nichtmetallischen Substratkörpers (Verfahrensschritt I);
b. Aufbringen der metallischen Schicht (53) der mehrschichtigen Beschichtung nach dem HiPIMS-Verfahren (Verfahrensschritt IV); und
c. Aufbringen der metalloxid- und/oder metallhydroxyapatithaltigen Schicht (51) auf die metallische Schicht (53) **durch** ein Sputterverfahren (Verfahrensschritt VI).

2. Verfahren nach Anspruch 1, **dadurch gekennzeichnet, dass** das Bereitstellen des nichtmetallischen Substratkörpers (55) (Verfahrensschritt I) die folgenden Schritte umfasst:
a.1 Erfassen von Daten eines Patienten, für welchen das Implantat oder die Osteosynthese bestimmt ist
a.2 Erstellen von Fertigungsvorgaben für das Implantat oder die Osteosynthese mittels eines Modells anhand der erfassten Daten;
a.3 Herstellung des Implantat- oder Osteosynthese-Substratkörpers auf Basis der Fertigungsvorgaben;

3. Verfahren nach Anspruch 2, **dadurch gekennzeichnet, dass** das Erstellen von Fertigungsvorgaben für zumindest zwei oder mehr zu einem Implantat und/oder zu einer Osteosynthese zusammensetzbare Teilelemente (1, 11, 12, 21, 26, 29) auf Grundlage des erstellten Modells erfolgt, wobei die Fertigungsvorgaben umfassen:
C1) eine Dimensionierung der Teilelemente (1, 11, 12, 21, 26, 29), wobei zumindest eines, vorzugsweise jedes, dieser Teilelemente (1, 11, 12, 21, 26, 29) als Substratkörper ausgebildet ist.

4. Verfahren nach einem der vorhergehenden Ansprüche, **dadurch gekennzeichnet, dass** das Bereitstellen des nichtmetallischen Substratkörpers (55) (Verfahrensschritt I) eine Oberflächenreinigung und/oder ein Oberflächenpolieren umfasst.

5. Verfahren nach einem der vorhergehenden Ansprüche, **dadurch gekennzeichnet, dass** nach dem Bereitstellen des nichtmetallischen Substratkörpers (55) (Verfahrensschritt I) und vor dem Aufbringen der metallischen Schicht (53) (Verfahrensschritt IV) eine physikalische oder physikalisch-chemische Vorbehandlung der Oberfläche des Implantat- oder Osteosynthese-Substratkörpers (55) (Verfahrensschritt II) durch eine Wärmebehandlung und/oder ein Ätzen erfolgt.

6. Verfahren nach einem der vorhergehenden Ansprüche, **dadurch gekennzeichnet, dass dadurch gekennzeichnet, dass** nach dem Bereitstellen des nichtmetallischen Substratkörpers (55) (Verfahrensschritt I) und vor dem Aufbringen der metallischen Schicht (53) (Verfahrensschritt IV) eine physikalische oder physikalisch-chemische Vorbehandlung der Oberfläche des Implantat- oder Osteosynthese-Substratkörpers (Verfahrensschritt II) durch Plasmaaktivierung und/oder durch Aktivierung mit einem Sauerstoffderivat und/oder einer radikalischen Verbindung erfolgt.

7. Verfahren nach einem der vorhergehenden Ansprüche, **dadurch gekennzeichnet, dass** nach dem Bereitstellen des nichtmetallischen Substratkörpers (55) (Verfahrensschritt I) und vor dem Aufbringen der metallischen Schicht (53) nach dem HiPIMS Verfahren (Verfahrensschritt IV) ein Abscheiden von Metall (54) auf dem Substratkörper (55) durch Sputtern, insbesondere durch Hochfrequenz-Sputtern, (Verfahrensschritt III) erfolgt.

8. Verfahren nach einem der vorhergehenden Ansprüche, **dadurch gekennzeichnet, dass** nach dem Aufbringen der metallischen Schicht (53) (Verfahrensschritt IV) ein Aufbringen einer porösen metallischen Schicht (52) (Verfahrensschritt V) insbesondere durch DC-Sputtern, erfolgt.

9. Verfahren nach einem der vorhergehenden Ansprüche, **dadurch gekennzeichnet, dass** nach dem Aufbringen der porösen metallischen Schicht (52) (Verfahrensschritt V) ein Aufbringen der metalloxid- und/oder metallhydroxyapatithaltigen Schicht (51) auf die poröse metallische Schicht (52) erfolgt oder eine Ausbildung der metalloxid- und/oder metallhydroxyapatithaltigen Schicht (52) auf der porösen metallischen Schicht (51) erfolgt.

10. Verfahren nach Anspruch 9, **dadurch gekennzeichnet, dass** die metalloxid- und/oder metallhydroxyapatithaltigen Schicht (51) durch ein DC-Reaktivsputtern (Verfahrensschritt VI) ausgebildet wird.

11. Implantat oder Osteosynthese umfassend einen Substratkörper (55) aus einem nichtmetallischen Material und eine zumindest dreischichtige Beschichtung (51-54), wobei eine metallische Schicht (53) dieser Beschichtung nach dem HiPIMS-Verfahren aufgebracht wurde.

12. Implantat oder Osteosynthese nach Anspruch 11, insbesondere hergestellt nach einem Verfahren gemäß einem der vorhergehenden Ansprüche 1-10, **dadurch gekennzeichnet, dass** eine Schicht der Beschichtung eine metalloxid- und/oder metallhydroxyapatithaltigen Schicht (51) ist.

13. Implantat oder Osteosynthese nach einem der vorhergehenden Ansprüche, **dadurch gekennzeichnet, dass** eine Schicht zwischen der metallischen Schicht (53), die nach dem HiPIMS-Verfahren aufgebracht wurde, und der metalloxid- und/oder metallhydroxyapatithaltigen Schicht (51) eine poröse metallische Schicht (52) ist mit einer Dichte, welche zwischen 50 bis 95% der theoretischen Dichte für das Metall dieser metallischen Schicht (52) liegt.

14. Implantat oder Osteosynthese nach einem der vorhergehenden Ansprüche, **dadurch gekennzeichnet, dass** der nichtmetallische Substratkörper (55) ein Kunststoffkörper ist, vorzugsweise ausgesucht aus der Klasse der Polyetherketone, der Polyethylene und/oder der Polyoxymethylene.

15. Implantat oder Osteosynthese nach einem der vorhergehenden Ansprüche, **dadurch gekennzeichnet, dass** das Metall der metallischen Schicht (53) und/oder der porösen metallischen Schicht (52) Titan und/oder Tantal ist und dass das Metalloxid und/oder das Metallhydroxyapatit eine Titan- und/oder Tantalverbindung ist.
